# EUROPÄISCHE PATENTSCHRIFT

(11) **EP 0 289 924 B1**
(45) Veröffentlichungstag und Bekanntmachung des Hinweises auf die Patenterteilung: **21.12.1994**
(21) Anmeldenummer: 88106707.8
(22) Anmeldetag: 27.04.1988
(51) Int. Cl.: C07D 213/08, C07D 213/14

(54) **Verfahren zur Herstellung von Pyridinen**
Process for the preparation of pyridines
Procédé pour la préparation de pyridines

(30) Priorität: 05.05.1987 DE 3714857
(43) Veröffentlichungstag der Anmeldung: 09.11.1988
(73) Patentinhaber: DEGUSSA AG, 60311 Frankfurt (DE)
(72) Erfinder: Hoelderich, Wolfgang, Dr., D-6710 Frankenthal (DE); Goetz, Norbert, Dr., D-6520 Worms 1 (DE)

(56) Entgegenhaltungen:
- EP-A- 0 131 887
- DE-A- 1 695 242
- DE-A- 2 038 533
- GB-A- 1 087 279
- US-A- 2 528 978
- US-A- 2 528 978
- US-A- 2 741 618

## Beschreibung

Die Erfindung betrifft ein Verfahren zur Herstellung von Pyridinen durch Umsetzung von 2-Alkoxy-2,3-dihydro-4H-pyranen oder Glutaraldehyden in Gegenwart von sauren, festen Zeolithkatalysatoren (Heterogenkatalysatoren).

Die GB-PS 1 102 261 beschreibt ein Verfahren zur Herstellung von Pyridinen durch Umsetzung von Glutaraldehyd, substituiertem Glutaraldehyd oder 2-Alkoxy-2,3-dihydro-4H-pyranen mit Ammoniak oder Ammoniumsalzen in Gegenwart von Sauerstoff und Kupfer-II- bzw. Eisen-III-halogeniden. Bezogen auf den eingesetzten Dialdehyd werden Pyridin-Selektivitäten von 44 % erzielt. Neben der mäßigen Ausbeute hat das Verfahren den Nachteil, daß bei der Reaktion, bezogen auf Aldehyd, 44 Mol-% Kupfer(II)chlorid und 542 Mol-% Ammoniumchlorid eingesetzt werden, die zu Nebenprodukten (Chlorpyridinen) führen, wodurch die Reindarstellung des nicht halogenierten Pyridins erschwert wird.

Aus EP-PS 57 366 ist bekannt, daß man die Reaktion mit Ammoniumnitrat in Gegenwart von aliphatischen Carbonsäuren ausführen kann. Dieses Verfahren hat den Nachteil, daß man den für die Reaktion notwendigen Ammoniak in Form von Ammoniumnitrat einsetzen, die Reaktion in Gegenwart der korrosiven Carbonsäuren ausführen und ein erhöhter Aufwand bei der Abtrennung des Wertproduktes aus dem Reaktionsgemisch geleistet werden muß. Auch die Verwendung von Salpetersäure als Kokatalysator ist ebenso wie die Bildung der salpetrigen Säure nachteilig, da beim Auftreten von Stickoxiden Vorsichtsmaßnahmen ergriffen werden müssen.

Auch ist bekannt, daß man Pyridine aus 2-Alkoxy-3,4-dihydropyranen an Oxidationskatalysatoren, z.B. Mo-Bi-P-Oxiden (JA 7244747-R) oder an mit Edelmetallen dotierten Al₂O₃ bzw. SiO₂ (GB 1 087 279), in Gegenwart von Wasser und Sauerstoff herstellen kann. Der hierzu notwendige Einsatz von Wasser und Sauerstoff erfordert einen erhöhten Aufwand an Energie (Aufheizen und Abkühlen des Wassers), bei der Abtrennung des Wertproduktes und an Sicherheitsmaßnahmen.

Es wurde nun gefunden, daß man Pyridine der Formel (I) 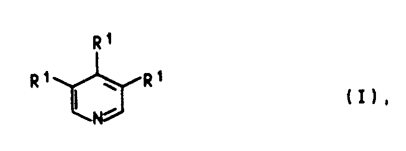
worin die einzelnen Reste R¹ gleich oder verschieden sein können und jeweils ein Wasserstoffatom oder einen aliphatischen Rest bedeuten, bei Vermeidung dieser Nachteile erhält, wenn man Ammoniak mit
a) 2-Alkoxy-2,3-dihydro-4H-pyrane der Formel (II) 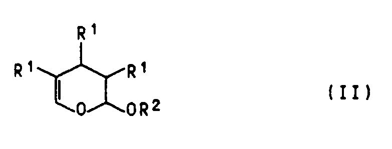 oder
b) Glutaraldehyde der Formel (III) 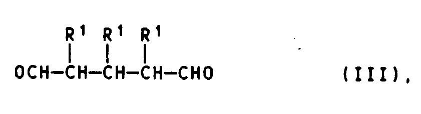 worin R¹ die vorgenannte Bedeutung besitzt und R² einen aliphatischen Rest bezeichnet, in Gegenwart von sauren, festen Zeolithkatalysatoren umsetzt.

Auch ist es im Prinzip möglich, daß die Alkoxygruppe statt in Position 1 auch in Position 2 oder 3 stehen kann.

Die Umsetzung kann für den Fall der Verwendung von 2,3-Dihydro-2-methoxy-4H-pyran oder Glutardialdehyd durch die folgenden Formeln wiedergegeben werden: 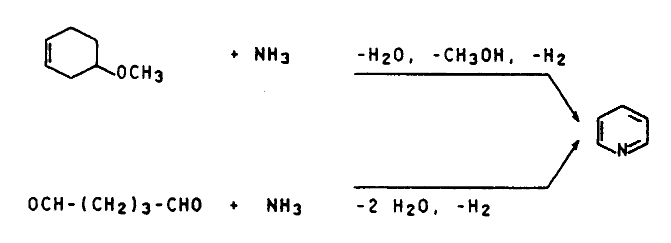
Im Vergleich zu den bekannten Verfahren liefert das erfindungsgemäße Verfahren auf einfacherem Wege Pyridine in besserer Raum-Zeit-Ausbeute, Ausbeute und Reinheit. Überraschenderweise kann dabei auf Sauerstoff und andere Oxidationsmittel, wie Salze des dreiwertigen Eisens oder Hangans oder des zweiwertigen Kupfers und auf Carbonsäuren verzichtet werden, woraus eine wesentlich vereinfachte Aufarbeitung sowie Isolierung des Reaktionsproduktes resultiert. Das erfindungsgemäße Verfahren ist außerdem umweltfreundlich, da zu seiner Durchführung keine abwasserbelastenden Schwermetallsalze benötigt werden und ohne korrosive Ausgangsstoffe gearbeitet wird.

Die Ausgangsstoffe (II) und (III) können mit Ammoniak in stöchiometrischer Menge oder im Überschuß, umgesetzt werden. Bevorzugte Ausgangsstoffe II und III und dementsprechend bevorzugte Endstoffe I sind solche, in deren Formeln die einzelnen Reste R¹ und R² gleich oder verschieden sein können und jeweils einen Alkylrest mit 1 bis 8 Kohlenstoffatomen bedeuten, wobei R¹ auch für ein Wasserstoffatom stehen kann. Die vorgenannten Reste können noch durch unter den Reaktionsbedingungen inerte Gruppen, z.B. Alkylgruppen, Alkoxygruppen oder Carboxygruppen mit jeweils 1 bis 4 Kohlenstoffatomen, substituiert sein.

So kommen beispielsweise folgende Ausgangsstoffe (II) in Betracht: In 3-, 4- oder 5-Stellung einfach oder in 3,4-, 4,5-, 3,5-Stellung gleich oder unterschiedlich zweifach oder in 3-, 4-, 5-Stellung gleich oder unterschiedlich dreifach durch Methyl-, Ethyl-, Propyl, Isopropyl, Butyl-, Isobutyl-, sek.-Butyl-, tert.-Butylgruppen substituierte 2,3-Dihydro-2-methoxy-4H-pyrane; homolog durch vorgenannte Substituenten substituierte oder unsubstituierte 2-Ethoxy-, 2-Propoxy-, 2-Isopropoxy-, 2-Butoxy-, 2-Isobutoxy-, 2-sek.-Butoxy-, 2-tert.-Butoxy-4H-pyrane.

Beispielsweise sind folgende Ausgangsstoffe (III) geeignet: In 2-, 3-, 4-Stellung einfach oder in 2,3-, 3,4-, 2,4-Stellung gleich oder unterschiedlich zweifach oder in 2,3,4-Stellung gleich oder unterschiedlich dreifach durch Methyl-, Ethyl-, Propyl-, Isopropyl-, Butyl-, Isobutyl-, sek. -Butyl-, tert.-Butylgruppen substituierte Glutaraldehyde; unsubstituierter Glutaraldehyd.

Als saure, feste Zeolithkatalysatoren (Heterogenkatalysatoren) für das erfindungsgemäße Verfahren werden acide zeolithische Katalysatoren eingesetzt. Zeolithe sind kristalline Aluminosilikate, die eine hochgeordnete Struktur mit einem starren dreidimensionalen Netzwerk von SiO₄- und AlO₄-Tetraedern besitzen, die durch gemeinsame Sauerstoffatome verbunden sind. Das Verhaltnis der Si- und Al-Atome zu Sauerstoff beträgt 1:2 (siehe Ullmanns Ecyclopädie der technischen Chemie, 4. Auflage, Band 24, Seite 575 (1983). Die Elektrovalenz der Aluminium enthaltenden Tetraeder ist durch Einschluß von Kationen in den Kristall, z.B. eines Alkali- oder Wasserstoffions, ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Metalle wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Fur das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind beschrieben in "Catalysis by Zeolites", Band 5 aus "Studies in Surface Science and Catalysis" ed. B. Imelik et al, Elsevier Scientific Publishing Comp. 1980, S. 203 und "Crystal Structures of Ultra-stable Faujasites" Advances in Chemistry Series Nr. 101, American Chemical Society Washington, DC, S. 226ff (1971) und in US-PS 4 512 961.

Besonders vorteilhaft verwendet man Zeolithe vom Pentasiltyp. Diese haben als Grundbaustein einen aus SiO₄-Tetraedern aufgebauten Fünfring gemeinsam. Sie sind durch ein hohes SiO₂/Al₂O₃-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeoltihe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise Al(OH)₃ oder Al₂(SO₄)₃ und einer Siliciumkomponente, vorzugswseise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Ausgangsstoffmengen ein SiO₂/Al₂O₃-Verhältnis von 10 bis 40 000. Derartige Aluminosilikatzeolithe kann man auch in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200°C unter autogenem Druck synthetisiert, indem man eine Borverbindung, z.B. H₃BO₃, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetraminlösung mit und insbesondere ohne Alkali- oder Erdalkalizusatz zur Reaktion bringt. Auch die obengenannten isotaktischen Zeolithe mit Bor gehören hierzu. Die Borosilikatzeolithe können ebenfalls statt in wäßriger Aminlösung auch in etherischer Lösung, z.B. Diethylenglykoldimethylether oder in alkoholischer Lösung, z.B. 1,6-Hexandiol, hergestellt werden.

Der Eisensilikatzeolith erhält man z.B. aus einer Eisenverbindung, vorzugsweise Fe₂(SO)₄)₃ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 220°C unter autogenem Druck.

Zu den verwendbaren siliciumreichen Zeolithen (SiO₂/Al₂O₃ ≧ 10) gehören auch die sog. ZSM-Typen, Ferrierit, NU-1 und Silicalite®-Molekularsiebe, sog. Silica Polymorphe.

Die so hergestellten Alumino-, Boro- und Eisensilikatzeolithe können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90:10 bis 40:60 Gew.% zu Strängen oder Tabletten verformt werden. Als Bindemittel eignen sich verschiedene Aluminiumoxide, bevorzugt Boehmit, amorphe Aluminosilikate mit einem SiO₂/Al₂O₃-Verhältnis von 25:75 bis 90:5, bevorzugt 75:25, Siliciumdioxid, bevorzugt hochdisperses SiO₂, Gemische aus hochdispersem SiO₂ und hochdispersem Al₂O₃, TiO₂, ZrO₂ sowie Ton. Nach der Verformung werden die Extrudate oder Preßlinge bei 110°C/16 h getrocknet und bei 500°C/16 h calciniert.

Man erhält auch vorteilhafte Katalysatoren, wenn der isolierte Alumino- bzw. Borosilikatzeolith direkt nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure. Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsatz sowie lange Standzeiten zu erreichen, kann es vorteilhaft sein, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetalle wie Mg, Ca, Sr. Metalle der 3., 4. und 5. Hauptgruppe wie Al, Ga, Ge, Sn, Pb, Bi, Übergangsmetalle der 4.-8. Nebengruppe wie Ti, Zr, V, Nb, Cr, Mo, W, Mn, Re, Fe, Ru, Os, Co, Rh, Ir, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man z.B. den verformten Zeolithen in einem Steigrohr vorlegt und bei 20 bis 100°C z.B. eine wäßrige oder ammoniakalische Lösung eines Halogenids oder eines Nitrats der voranbeschriebenen Metalle überleitet. Ein derartiger Ionenaustausch kann z.B. an der Wasserstoff-, Ammonium- und Alkaliform des Zeolithen vorgenommen werden. Eine weitere Möglichkeit der Metallaufbringung auf den Zeolithen ist gegeben, indem man das zeolithische Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der verformte oder unverformte Zeolith eine gewisse Zeit, etwa. 30 Minuten, getränkt. Die eventuell überstehende Losung wird am Rotationsverdampfer von Wasser befreit. Danach wird der getränkte Zeolith bei etwa 150°C getrocknet und bei etwa 550°C calciniert. Dieser Tränkvorgang kann mehrmals hintereinander vorgenommen werden, um den gewünschten Metallgehalt einzustellen.

Es ist auch möglich, eine wäßrige Ni(NO₃)₂-Lösung oder ammoniakalische Pd(NO₃)₂-Lösung herzustellen und darin den reinen pulverförmigen Zeolithen bei 40 bis 100°C unter Rühren etwa 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form, Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten Zeolithen z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft z.B. so vor, daß man Zeolithe in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert. Nach einer anderen Arbeitsweise behandelt man Zeolithe vor oder nach ihrer Verformung mit Bindemitteln, z.B. 1 bis 3 Stunden bei Temperaturen von 60 bis 80°C mit einer 3 bis 25 gew.%igen, insbesondere 12 bis 20 gew.%igen wäßrigen Salzsäure. Anschließend wird der so behandelte Zeolith mit Wasser gewaschen, getrocknet und bei 400 bis 500°C calciniert.

Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von etwa 0,5 bis 5, vorzugsweise 1 bis 3 Stunden. Nach Isolierung, z.B. durch Abfiltrieren und Auswaschen, des zeolithischen Materials wird dieses zweckmäßig bei Temperaturen von 100 bis 160°C getrocknet und bei Temperaturen von etwa 450 bis 600°C calciniert. Gemäß einer weiteren bevorzugten Ausführungsform für die Saurebehandlung wird das zeolithische Material nach einer Verformung mit Bindemittel bei erhöhter Temperatur, zweckmäßig bei Temperaturen von 50 bis 90°C. vorzugsweise 60 bis 80°C, über einen Zeitraum von 0,5 bis 5 Stunden, vorzugsweise 1 bis 3 Stunden, mit 1 bis 30 gew.%iger, vorzugsweise mit 12 bis 20 gew.%iger Salzsäure, behandelt. Anschließend wird das zeolithische Material ausgewaschen und zweckmäßig bei Temperaturen von 100 bis 160°C getrocknet und bei Temperaturen von etwa 450 bis 600°C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethylphosphat, Trimethoxyphosphin, primärem, sekundärem oder tertiärem Natriumphosphat modifizieren. Hierbei werden die Zeolithe in Strang-, Tabletten- oder Wirbelgut-Form, z.B. mit wäßriger NaH₂PO₄-Lösung, getränkt, bei 110°C getrocknet und bei 500°C calciniert.

Wenn bei der erfindungsgemäßen Verwendung der zeolithischen Katalysatoren wie auch der anderen hierfür eingesetzten Katalysatoren eventuell eine durch Koksabscheidung bedingte Desaktivierung eintritt, empfiehlt es sich, diese durch Abbrennen der Koksablagerung mit Luft oder mit einem Luft/N₂-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2- bis 4-mm-Stränge oder als Tabletten mit 3 bis 5 mm Durchmesser oder als Splitt mit Teilchengroßen von 0,1 bis 0,5 mm als Wirbelkontakt eingesetzt werden.

Das erfindungsgemäße Verfahren kann unter folgenden Reaktionsbedingungen ausgeführt werden:

Das Molverhältnis von Ausgangsstoff II bzw. III:NH₃ kann 1:1 bis 10, insbesondere 1:1 bis 5 molar betragen.

Die Umsetzung wird vorteilhaft in der Gasphase, z.B. bei Temperaturen von 100 bis 500°C, vorteilhaft bei 150 bis 450°C, insbesondere bei 200 bis 400°C, ausgeführt. In der Regel führt man die Umsetzung bei einem Druck von 0,1 bis 100 bar, insbesondere 0,5 bis 10 bar durch.

In der Gasphase wird zweckmäßig eine Katalysatorbelastung von 0,1 bis 20, insbesondere von 1 bis 10 g Ausgangsstoff je g Katalysator und Stunde (WHSV = g Ausgangsstoff/g Katalysator und Stunde) gewählt.

Die Reaktionen in der Gasphase können in einem Festbett oder in einem Wirbelbett ausgeführt werden.

Es ist auch möglich, die Reaktion in der Flüssigphase (Suspension-, Riesel- oder Sumpffahrweise) bei Temperaturen zwischen 50 und 200°C durchzuführen.

Man kann kontinuierlich, aber auch diskontinuierlich arbeiten.

Schwerflüchtige oder feste Ausgangsstoffe werden in gelöster Form, z.B. in THF-, Toluol- oder Petrolether-Losung eingesetzt. Im allgemeinen ist eine Verdünnung des Ausgangsstoffes mit derartigen Lösungsmitteln oder mit Inertgasen wie N₂, Ar, H₂O-Dampf möglich. Auch ist es möglich, die Reaktion in Gegenwart von O₂ auszuführen.

Nach der Umsetzung werden die entstandenen Produkte durch übliche Verfahren, z.B. durch Destillation, aus dem Reaktionsgemisch isoliert; nichtumgesetzte Ausgangsstoff werden gegebenenfalls in die Umsetzung zurückgeführt.

Die nach dem erfindungsgemäßen Verfahren erhältlichen substituierten Pyridine sind vielseitig verwendbare Zwischenprodukte für die Herstellung von Farbstoffen, Pharmazeutika, Schädlingsbekämpfungsmitteln und sind wertvolle Lösungsmittel. Anwendungsmöglichkeiten sind beschrieben in Chem. Techn. 19 (9), S. 528-537 (1967) und Ullmanns Enzyklopädie der technischen Chemie, Band 14, S. 467.

### Beispiele 1-15

Die Reaktionen in der Gasphase werden unter isothermen Bedingungen in einem Rohrreaktor (Wendel, 0,6 cm Innendurchmesser, 90 cm Länge) mindestens 6 Stunden lang durchgeführt. Die Reaktionsprodukte werden durch übliche Methoden abgetrennt und charakterisiert. Die quantitative Bestimmung der Reaktionsprodukte und der Ausgangsstoffe erfolgt gaschromatographisch nach bekannter Methode.

Es werden folgende Katalysatoren verwendet:

### Katalysator A

Der Borosilikatzeolith des Pentasil-Typs wird in einer hydrothermalen Synthese aus 640 g hochdispersem SiO₂, 122 g H₃BO₃, 8 000 g einer wäßrigen 1,6-Hexandiamin-Lösung (Mischung 50:50 Gew.-% bei 170°C unter autogenem Druck in einem Rührautoklaven hergestellt. Nach Abfiltrieren und Auswaschen wird das kristalline Reaktionprodukt bei 100°C/24 h getrocknet und bei 500°C/24 h calciniert. Dieser Borosilikatzeolith setzt sich zusammen aus 94,2 Gew.-% SiO₂ und 2,3 Gew.-% B₂O₃.

Katalysator A wird erhalten, indem man den Borosilikatzeolithen mit Boehmit im Gewichtsverhältnis 60:40 zu 2-mm-Strängen verformt, bei 110°C/16 h trocknet und bei 500°C/24 h calciniert.

### Katalysator B

Ein Aluminosilikatzeolith vom Pentasil-Typ wird unter hydrothermalen Bedingungen bei autogenem Druck und 150°C aus 65 g hochdispersem SiO₂, 20,3 g Al₂(SO₄)₃ x 18 H₂O in 1 kg einer wäßrigen 1,6-Hexandiamin-Lösung (Mischung 50:50 Gew.-%) in einem Rührautoklaven hergestellt. Nach Abfiltrieren und Auswaschen wird das kristalline Reaktionprodukt bei 110°C/24 h getrocknet und bei 500°C/24 h calciniert. Dieser Aluminosilikatzeolith enthält 91,6 Gew.-% SiO₂ und 4,6 Gew.-% Al₂O₃.

Katalysator B wird erhalten, indem man den Aluminiumsilikatzeolithen mit Boehmit im Gewichtsverhältnis 60:40 zu 2-mm-Strängen verformt, bei 100°C/16 h trocknet und bei 500°C/24 h calciniert.

### Katalysator C

100 g des bei Katalysator A verwendeten Borosilikatzeolithen werden mit 280 ml einer 0,1 n HF bei 90°C 2 h lang behandelt und nach Abfiltrieren bei 160°C getrocknet. Dieses Produkt wird mit amorphem Aluminosilikat (25 Gew.% Al₂O₃ und 75 Gew.% SiO₂) im Gewichtsverhältnis 60:40 zu 2-mm-Strängen verformt, bei 110°C/16 h getrocknet und bei 500°C/16 h calciniert.

### Katalysator D

Katalysator D wird erhalten, indem man Katalysator A mit einer wäßrigen La(NO₃)₂-Lösung imprägniert, danach bei 130°C/2 h trocknet und bei 540°C/2 h calciniert. Der La-Gehalt beträgt 3,2 Gew.%.

### Katalysator E

Mit dem bei Katalysator A hergestellten Borosilikatzeolithpulver werden durch Verformen mit einem Verformungshilfsmittel 2-mm-Stränge hergestellt, die bei 110°C/16 h getrocknet und bei 500°C/24 h calciniert werden. Katalysator E wird erhalten, indem man diese Stränge mit einer wäßrigen Ce(NO₃)₃-Lösung imprägniert, bei 130°C/2 h trocknet und bei 540°C/2 h calciniert. Der Ce-Gehalt beträgt 2,5 Gew.%.

### Katalysator F

Katalysator F wird erhalten wie Katalysator E, indem man jedoch Co(NO₃)₂-Lösung statt Ce(NO₃)₃-Lösung verwendet. Der Co-Gehalt beträgt 3,2 Gew.%.

### Katalysator G

Katalysator G wird erhalten, indem man Katalysator B mit einer wäßrigen La(NO₃)₃-Lösung imprägniert, bei 130°C/2 h trocknet und bei 540°C/2 h calciniert. Der La-Gehalt beträgt 3,2 Gew.%.

### Katalysator H

Handelsüblicher NaY-Zeolith wird mit Boehmit im Gewichtsverhältnis 60:40 zu 2-mm-Strängen verformt, bei 110°C/16 h getrocknet unde bei 540°C/24 h calciniert. Diese Stränge werden mit einer 20 %igen wäßrigen La(NO₃)₂-Lösung bei 80°C/2 h ionenausgetauscht. Nach Trocknung bei 110°C und Calcination bei 500°C soll der La-Gehalt 7,1 Gew.% und der Na-Gehalt 1,1 Gew.% betragen. Der Ionenaustausch kann nach Zwischencalcination wiederholt werden bis obige La- bzw. Na-Gehalte eingestellt sind.

### Vergleichskatalysator I

660 g Kieselsol (30 Gew.% SiO₂) werden mit 567 g wäßriger Tetrapropylammoniumhydroxid-Losung (20 %ig) gemischt und in einem Autoklaven bei 200°C 72 h umgesetzt. Nach Abtrennen von der Mutterlauge wird bei 120°C getrocknet und bei 500°C/16 h calciniert. Das Diffraktogramm zeigt die für Silicalit® typischen Röntgenbeugungsmuster. Dieser Silicalit wird mit hochdispersem SiO₂ im Gewichtsverhältnis 70:30 zu 2-mm-Strängen verformt. Diese Stränge weden mit einer 20 %igen NH₄Cl-Lösung bei 80°C in einer Kolonne ionenausgetauscht. Danach wird mit Wasser ausgewaschen, bei 110°C getrocknet und bei 500°C/5 h calciniert. Der Na-Gehalt des Silicaliten beträgt nach dieser Verfahrensweise 0,015 Gew.%. Der Ionenaustausch kann wiederholt werden, um diesen Na-Gehalt einzustellen.

### Katalysator J

Der Eisensilikatzeolith des Pentasil-Typs wurde unter hydrothermalen Bedingungen bei autogenem Druck und 165°C aus 273 g Wasserglas, gelöst in 253 g einer wäßrigen 1,6-Hexandiamin-Lösung (Mischung 50:50 Gew.%) und 31 g Eisensulfat, gelöst in 21 g 96 %iger Schwefelsäure und 425 g Wasser in einem Rührautoklaven während 4 Tagen synthetisiert. Der Zeolith wurde abfiltriert, ausgewaschen, bei 110°C/24 h getrocknet und bei 500°C/24 h calciniert. Man erhielt einen Eisensilikatzeolith mit einem SiO₂/Fe₂O₃-Verhältnis von 17,7 und einen Na₂O-Gehalt von 1,2 Gew.%. Der Katalysator wurde mit hochdispersem SiO₂ im Gewichtsverhältnis 80:20 zu 2,5-mm-Strängen verstrangt, bei 110°C/16 h getrocknet und bei 500°C/24 h calciniert.

Die mit diesen Katalysatoren erzielten Versuchsergebnisse und die Versuchsbedingungen sind in den Tabellen 1 und 2 beschrieben.

Aus den Tabellen 1 und 2 erkennt man, daß die zeolithischen Katalysatoren besonders vorteilhaft für das erfindungsgemäße Verfahren sind.

Nebenprodukte bei diesen Reaktionen sind oft Methylpyridine und Ethylpyridine.

**Tabelle 1**

| Pyridin aus 2-Methoxy-2,3-dihydro-4H-pyran und NH₃ (molares Verhältnis 1:3) | | | | | | | |
|---|---|---|---|---|---|---|---|
| Beispiel | 1 | 2 | 3 | 4 | 5 | 6 | 7 |
| Katalysator | A | B | C | E | H | I | J |
| Temperatur °C | 400 | 400 | 400 | 400 | 400 | 400 | 400 |
| WHSV h⁻¹ | 3 | 3 | 3 | 3 | 3 | 3 | 3 |
| Umsatz % | 100 | 100 | 100 | 100 | 100 | 100 | 100 |
| Selektivität % Pyridin | 93,1 | 93,9 | 95,3 | 76,8 | 78,6 | 77,4 | 80,3 |

**Tabelle 2**

| Pyridin aus Glutaraldehyd und NH₃ | | | | | | | | |
|---|---|---|---|---|---|---|---|---|
| Beispiel | 8 | 9 | 10 | 11 | 12 | 13 | 14 | 15 |
| Katalysator | A | B | C | D | E | F | G | H |
| Temperatur °C | 400 | 400 | 400 | 400 | 400 | 400 | 400 | 400 |
| WHSV h⁻¹ | 1,0 | 0,5 | 0,5 | 0,5 | 0,5 | 0,5 | 0,5 | 0,5 |
| Umsatz % | 100 | 100 | 100 | 100 | 100 | 100 | 100 | 100 |
| Selektivität % Pyridin | 84,1 | 83,0 | 88,7 | 80,1 | 78,6 | 84,9 | 75,3 | 88,5 |
| 1) bezogen auf Glutaraldehyd 2) molares Verhältnis Glutaraldehyd:NH₃ = 1:6 3) 25 %ige wäßrige Glutaraldehyd-Lösung | | | | | | | | |

### Beispiele 16 und 17

Lösung aus 4-Phenyl-2-isopropyloxydihydropyran und THF im Gewichtsverhältnis 9:1 wird im isothermen Festbettreaktor (siehe vorangehende Beispiele) mit NH₃ im molaren Verhältnis 1:32 bei 400°C und WHSV = 2,5 h⁻¹ an Katalysator H umgesetzt. Der Umsatz am Dihydropyran beträgt 63,3 % und die Selektivität an Phenylpyridin 88,9 %.

Am Katalysator A erhält man unter gleichen Bedingungen 62,5 % Umsatz und 79.7 % Selektivität.

### Beispiele 18 und 19

Losung aus 3-Hexyl-2-butoxydihydropyran und THF im Gewichtsverhältnis 50:50 wird im isothermen Festbettreaktor mit NH₃ im molaren Verhältnis 1:3 bei 350°C und WHSV = 2 h⁻¹ an Katalysator A umgesetzt. Der Umsatz des Dihydropyrans beträgt 100 % und die Selektivität an 3-Hexylpyridin 74,3 %.

Am Katalysator U erhält man unter gleichen Bedingungen 100 % Umsatz und 78,2 % Selektivität.

## Patentansprüche

1. Verfahren zur Herstellung von Pyridinen der Formel (I) 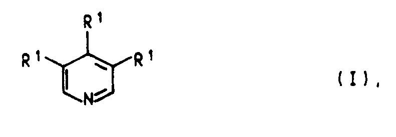 worin die einzelnen Reste R¹ gleich oder verschieden sein können und jeweils ein Wasserstoffatom oder einen aliphatischen Rest bedeuten, dadurch gekennzeichnet, daß man
a) 2-Alkoxy-2,3-dihydro-4H-pyrane der Formel (II) 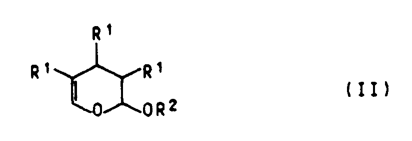 oder
b) Glutaraldehyde der Formel (III) 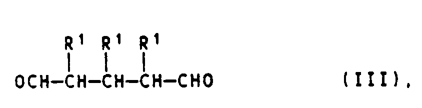 worin R¹ die vorgenannte Bedeutung besitzt und R² einen aliphatischen Rest bezeichnet, mit Ammoniak in Gegenwart von sauren, festen Zeolith-Katalysatoren umsetzt.

2. Verfahren nach Anspruch 1, dadurch gekennzeichnet, daß man als Katalysatoren Zeolithe des Pentasiltyps verwendet.

3. Verfahren nach den Ansprüchen 1 bis 2, dadurch gekennzeichnet, daß man als Katalysatoren Borosilikatzeolithe des Pentasiltyps verwendet.

4. Verfahren nach den Ansprüchen 1 bis 2, dadurch gekennzeichnet, daß man als Katalysatoren Eisensilikatzeolithe des Pentasiltyps verwendet.

5. Verfahren nach den Ansprüchen 1 bis 2, dadurch gekennzeichnet, daß man als Katalysatoren Aluminiumsilikatzeolithe des Pentasiltyps verwendet.

6. Verfahren nach Anspruch 1, dadurch gekennzeichnet, daß man als Katalysatoren Aluminiumsilikatzeolithe des Faujasittyps verwendet.

7. Verfahren nach den Ansprüchen 1 bis 6, dadurch gekennzeichnet, daß die Katalysatoren mit Alkalimetallen und/oder mit Erdalkalimetallen und/oder mit Übergangsmetallen und/oder mit Seltenen Erdmetallen dotiert sind.

8. Verfahren nach den Ansprüchen 1 bis 7, dadurch gekennzeichnet, daß die Katalysatoren mit Säuren behandelt sind.

9. Verfahren nach Anspruch 8, dadurch gekennzeichnet, daß die Katalysatoren mit HF und/oder HCl behandelt sind.

10. Verfahren noch den Ansprüchen 1 bis 9, dadurch gekennzeichnet, daß die Reaktionen in der Gasphase ausgeführt werden.

## Claims

1. Process for the preparation of pyridines of the formula (I) 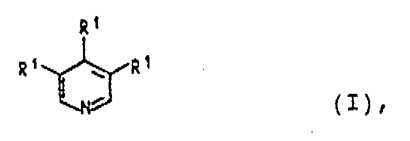 where the individual radicals R¹ may be identical or different and are each a hydrogen or an aliphatic rest, characterised in that
a) 2-alkoxy-2, 3-dihydro-4H-pyranes of the formula (II) 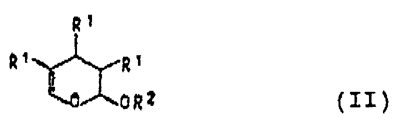 or
b) glutaraldehyde of the formula (III) 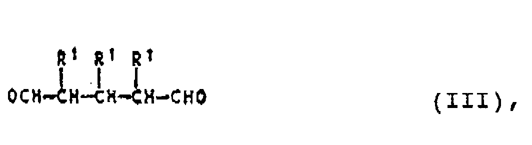 where R¹ has the above-mentioned meanings and R² is an aliphatic rest, is reacted with ammonia in the presence of acidic, solid zeolite catalysts.

2. The process of claim 1, wherein the catalysts used are zeolites of the pentasil type.

3. The process of claims 1 to 2, wherein in the catalysts used are borosilicate zeolites of the pentasil type.

4. The process of claims 1 to 2, wherein the catalysts used are iron silicate zeolites of the pentasil type.

5. The process of claims 1 to 2, wherein the catalysts used are aluminosilicate zeolites of the pentasil type.

6. The process of claim 1, wherein the catalysts used are aluminosilicate zeolites of the faujasite type.

7. The process of claims 1 to 6, wherein the catalysts are treated with alkaline metals and/or with alkaline-earth metals and/or with transition metals and/or with rare earth metals.

8. The process of claims 1 to 7, wherein the catalysts are treated with acids.

9. The process of claim 8, wherein the catalysts are treated with HF and/or HCl.

10. The process of claims 1 to 9, wherein the reactions are carried out in the gas phase.

## Revendications

1. Procédé de préparation de pyridines de la formule (I) 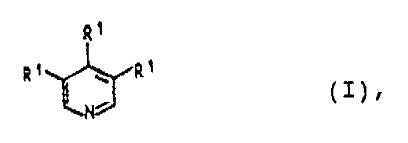 où les restes individuels R¹ sont identiques ou différents et où chacun est un hydrogène ou on rest aliphatique caractérisé en ce qu'on fait réagir
a) (2-alcoxy-2, 3-dihydro-4H-pyranes) de la formule (II) 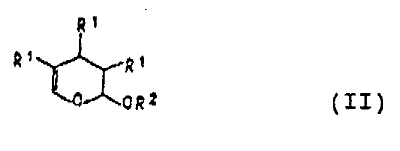 ou
b) aldéhydes de la formule (III) 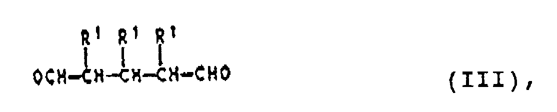 où R¹ a les significations données ci-dessus et R² est un rest aliphatique, avec ammoniac que en présence d'un catalyseur acide du type zéolite.

2. Procédé suivant la revendication 1, caractérisé en ce qu'on utilise, comme catalyseurs, des zéolites du type pentasil.

3. Procédé suivant les revendications 1 à 2, caractérisé en ce qu'on utilise, comme catalyseurs, des silicates du bore zéolites du type pentasil.

4. Procédé suivant les revendications 1 à 2, caractérisé en ce qu'on utilise, comme catalyseurs, des silicates du fer zéolites du type pentasil.

5. Procédé suivant les revendications 1 à 2, caractérisé en ce qu'on utilise, comme catalyseurs, des silicates d'aluminium zéolites du type pentasil.

6. Procédé suivant la revendication 1, caractérisé en ce qu'on utilise, comme catalyseurs, des silicates d'aluminium zéolites du type faujasite.

7. Procédé suivant les revendications 1 à 6, caracterisé en ce que les catalyseurs utilisés sont traités avec des métaux alcalins et/ou avec les métaux alcalino-terreux et/ou avec des métaux transitoires et/ou avec des métaux terreux rares.

8. Procédé suivant les revendications 1 à 7, caractérisé en ce que les catalyseurs utilisés sont traités avec des acides.

9. Procédé suivant la revendication 8, caractérisé en ce que les catalyseurs utilisés sont traités avec HF et/ou HCl.

10. Procédé suivant les revendications 1 à 9, caractérisé en ce que les réactions are effectués dans la phase gazeuse.
